# FASCICULE DE BREVET EUROPEEN

(11) **EP 0 474 648 B1**
(45) Date de publication et mention de la délivrance du brevet: **21.08.1996**
(21) Numéro de dépôt: 90906785.2
(22) Date de dépôt: 25.05.1990
(51) Int. Cl.: C07C 2/72, B01J 23/04, B01J 31/12

(54) **PROCEDE POUR LA SYNTHESE D'HYDROCARBURES AROMATIQUES ALKYLES**
VERFAHREN ZUR HERSTELLUNG VON ALKYLIERTEN AROMATISCHEN KOHLENWASSERSTOFFEN
PROCESS FOR THE SYNTHESIS OF AROMATIC ALKYLATED HYDROCARBONS

(30) Priorité: 26.05.1989 FR 8907053
(43) Date de publication de la demande: 18.03.1992
(73) Titulaire: SOLVAY INTEROX (Société Anonyme), B-1050 Bruxelles (BE)
(72) Inventeur: GANHY, Jean-Pierre, B-1170 Bruxelles (BE); JACOBS, Pierre, B-1686 Gooik (BE); BAES, Marleen, B-3030 Heverlee (BE); MARTENS, Johan, B-1090 Bruxelles (BE)
(74) Mandataire: Decamps, Alain René François
(86) Numéro de dépôt international: BE9000022
(87) Numéro de publication internationale: WO9014323

(56) Documents cités:
- GB-A- 1 259 535

## Description

La présente invention concerne un procédé pour la fabrication d'hydrocarbures aromatiques alkylés. Elle concerne plus particulièrement la fabrication d'hydrocarbures aromatiques alkylés à chaîne alkyle comprenant au moins quatre atomes de carbone par condensation d'un hydrocarbure aromatique substitué par un groupement alkyle à chaîne courte comportant 1 à 3 atomes de carbone avec une oléfine en présence d'un catalyseur.

On connaît depuis longtemps un procédé de synthèse de composés aromatiques alkylés consistant à condenser un hydrocarbure aromatique substitué par un groupe comportant un atome de carbone saturé lié à au moins un atome d'hydrogène avec un hydrocarbure insaturé (brevet US-2758140 (V.N. Ipatieff et al.) colonne 2, lignes 19 à 45 et colonne 23, revendication 1). La réaction peut s'effectuer en présence d'agents de contact parmi lesquels on trouve l'alumine.

Ce procédé connu présente toutefois l'inconvénient de donner lieu à des sélectivités faibles et à des rendements en hydrocarbure aromatique alkylé peu élevés (colonnes 5 et 6, tableau 1).

Dans le brevet britannique GB-1259535, il est décrit un procédé d'alkylation d'hydrocarbures aromatiques en présence d'un catalyseur contenant un métal alcalin ou un hydrure de métal alcalin déposé sur de l'alumine. Le catalyseur est préparé préalablement à la réaction d'alkylation par mélange du métal avec l'alumine sous atmosphère d'azote suivi d'un traitement thermique à 300 °C pendant plusieurs heures (page 2, lignes 15 à 20 et lignes 38 à 44). Dans la demande de brevet européen EP-A1-0328940, il est divulgué un procédé similaire de synthèse d'hydocarbures aromatiques alkylés où l'on prépare aussi le catalyseur dans une étape préliminaire consistant à traiter à une température de 200 à 600 °C une alumine avec un métal alcalin et un hydroxyde de métal alcalin en présence de petites quantités d'humidité et dans une atmosphère gazeuse inerte (revendication 1 page 12).

Ces procédés présentent le désavantage de nécessiter une étape supplémentaire de préparation du catalyseur immédiatement avant son utilisation dans la réaction d'alkylation. Outre les coûts supplémentaires liés à l'appareillage et aux calories nécessaires au traitement thermique, ces procédés impliquent des difficultés de manutention des catalyseurs, ces derniers ayant généralement l'inconvénient d'être pyrophoriques.

L'invention entend remédier à ces inconvénients en fournissant un procédé qui permette d'obtenir des sélectivités proches de 100 % avec des rendements en hydrocarbure aromatique alkylé qui dépassent 90 % sans nécessiter la préparation préalable du catalyseur.

A cet effet, l'invention concerne un procédé de synthèse d'hydrocarbures aromatiques alkylés à chaîne alkyle saturée comprenant au moins quatre atomes de carbone selon lequel on fait réagir un hydrocarbure aromatique substitué par un groupement alkyle à chaîne courte saturée comportant un à trois atomes de carbone avec une oléfine en présence d'un catalyseur constitué d'au moins un métal alcalin ou un hydrure de métal alcalin imprégné sur un support d'alumine ; selon l'invention, on prépare le catalyseur dans le milieu réactionnel en présence de l'hydrocarbure aromatique à courte chaîne alkyle par mélange de l'alumine anhydre avec le métal alcalin ou avec l'hydrure de métal alcalin, puis on introduit l'oléfine pour démarrer la réaction.

L'invention vise à préparer des hydrocarbures aromatiques alkylés par une chaîne alkyle saturée linéaire ou ramifiée comprenant plus de quatre et, de préférence, pas plus de huit atomes de carbone. Elle s'adresse en particulier à la préparation d'hydrocarbures benzéniques alkylés par une chaîne alkyle saturée et ramifiée comprenant cinq atomes de carbone. Elle est tout particulièrement adaptée à la préparation d'amylbenzènes comme, par exemple, le tert-amylbenzène.

Par hydrocarbure aromatique alkylé à chaîne courte utilisé comme matière première selon l'invention, on entend désigner tout hydrocarbure aromatique comportant un ou plusieurs cycles aromatiques à cinq ou six atomes de carbone et au moins une chaîne alkyle latérale saturée comprenant un à trois atomes de carbone. Les hydrocarbures benzéniques à un seul noyau aromatique sont préférés et tout particulièrement le toluène, les diméthyl et triméthylbenzène, l'éthylbenzène, les éthyltoluènes, le n-propyl-benzène et le cumène.

L'oléfine utilisée comme agent d'alkylation doit comporter une chaîne carbonée linéaire comprenant au moins deux et pas plus de cinq atomes de carbone, deux au moins d'entre eux étant liés par une liaison insaturée de type oléfinique. Les monooléfines ne présentant qu'une seule insaturation sont préférées. A titre d'exemples, les oléfines suivantes peuvent être utilisées dans le procédé selon l'invention : l'éthylène, le propylène, les n-butènes, l'isobutène, le butadiène, les n-pentènes et les méthylbutènes.

Le catalyseur mis en oeuvre dans le procédé selon l'invention doit comporter au moins un métal alcalin ou un hydrure de métal alcalin en plus du support d'alumine. Tout métal alcalin peut convenir. Le sodium ou l'hydrure de sodium sont cependant généralement préférés en raison de leur bonne activité et de leur disponibilité élevée. Le sodium peut être le seul métal alcalin présent avec le support d'alumine; un tel catalyseur a donné d'excellents résultats.

Une variante du catalyseur conforme au procédé selon l'invention consiste à mettre en oeuvre un support d'alumine et un mélange de métaux alcalins ou d'hydrures de métaux alcalins dans des proportions sensiblement égales ou, dans une autre variante, telles qu'un seul des métaux alcalins ou des hydrures de métaux alcalins n'excède jamais le double de la proportion de tous les autres métaux alcalins ou hydrures de métaux alcalins réunis.

Une autre variante particulièrement intéressante du catalyseur conforme au procédé selon l'invention consiste à mélanger à un métal alcalin principal ou à un hydrure de métal alcalin de petites quantités n'excédant pas quelques pour cent d'un ou de deux autres métaux alcalins ou hydrures de métaux alcalins à titre de promoteur et de les additionner au support d'alumine. Le mélange de sodium ou d'hydrure de sodium avec de 0,1 à 5 % de rubidium et/ou de césium ou d'hydrure de rubidium et/ou de césium est tout particulièrement digne d'intérêt.

Il est aussi possible de combiner dans un même catalyseur un métal alcalin avec un hydrure de métal alcalin.

Les quantités de métal alcalin ou d'hydrure de métal alcalin mises en oeuvre sont le plus souvent telles que le catalyseur présente un rapport pondéral métal alcalin/alumine ou hydrure de métal alcalin/alumine compris entre 0,02 et 0,50 et, de préférence, entre 0,08 et 0,20.

Selon l'invention, le support d'alumine peut être constitué d'alumine de différentes variétés cristallines pures ou en mélange. Les variétés d'alumine α, β et γ conviennent bien. De bons résultats ont été obtenus avec un support constitué d'alumine γ pure.

Les alumines utilisées comme support du catalyseur dans le procédé selon l'invention sont des substances poreuses. Elles présentent généralement un diamètre moyen de pores compris entre 0,1 et 500 nm. Des résultats intéressants ont été obtenus avec des alumines dont le diamètre moyen des pores se situait entre 2 et 100 nm. Les meilleurs résultats ont été obtenus en mettant en oeuvre des alumines de 20 et 30 nm de diamètre moyen de pores.

La surface spécifique de ces alumines doit aussi se trouver dans la plage allant de 10 à 360 m²/g d'alumine pour que le catalyseur de métal alcalin mélangé avec ces alumines soit efficace. En pratique, on préfère les alumines dont la surface spécifique est comprise entre 50 et 200 m²/g d'alumine.

Généralement, les alumines qui conviennent pour accompagner les métaux alcalins dans les catalyseurs conformes au procédé selon l'invention présentent un volume de pores compris entre 25 et 65 ml/100 g d'alumine et, de préférence, entre 45 et 60 ml/100 g d'alumine.

Les alumines mises en oeuvre doivent être exemptes de traces d'eau libre. Au besoin, il peut être utile de leur faire subir préalablement à leur mise en oeuvre comme support du métal alcalin une calcination à une température comprise entre 200 et 600°C dans le but d'éliminer toute trace d'humidité résiduelle.

Les quantités de catalyseur optimales ne sont pas critiques. Pour assurer une bonne efficacité du catalyseur, il convient cependant que le rapport pondéral du métal alcalin à l'hydrocarbure aromatique à courte chaîne soit situé dans la plage allant de 0,0015 à 0,03.

Une variante intéressante du procédé selon l'invention consiste à ajouter de l'hydroxyde de potassium lors de la préparation du catalyseur dans le milieu réactionnel. Les quantités d'hydroxyde de potassium ajoutées se situent généralement en quantité telle que le rapport pondéral hydroxyde de K / métal alcalin ou hydrure de métal alcalin est compris entre 0,5 et 2 et, de préférence, entre 0,8 et 1,5.

La température et la pression auxquelles on effectue la réaction d'alkylation conforme au procédé selon l'invention doivent être adaptées à la nature de l'oléfine et de l'hydrocarbure aromatique de départ. On a cependant constaté que des pressions situées dans la plage de 0,5 à 10 MPa et des températures comprises entre 110 et 250°C conviennent bien. Des pressions de 1 à 5 MPa et des températures de 170 à 190°C ont donné les meilleurs résultats. Lorsque l'on effectue la réaction en phase vapeur, il est avantageux de fixer les pressions respectives de l'oléfine et de l'hydrocarbure aromatique de départ de manière telle que les rapports molaires oléfine/hydrocarbure aromatique soient compris dans la gamme allant de 0,7 à 1,3 et, de préférence, voisins de 1.

Afin d'assurer un bon contact entre les réactifs, il est généralement nécessaire d'effectuer la réaction sous une agitation vigoureuse, en particulier lorsque l'hydrocarbure de départ est liquide et l'oléfine est gazeuse dans les conditions de la réaction. Tous les types d'agitateurs connus conviennent généralement bien. Le choix d'un agitateur particulier s'effectue en fonction du type d'appareillage utilisé pour le réacteur.

La réaction conforme au procédé selon l'invention peut indifféremment s'effectuer dans un réacteur continu ou discontinu. Lorsque la réaction met en oeuvre un système à deux phases différentes, les réacteurs continus peuvent être choisis parmi les réacteurs classiques tels que les réacteurs à lit fixe, les lits fluidisés ou les réacteurs à lit mobile permettant la circulation et la régénération éventuelle du catalyseur. Si trois phases sont présentes simultanément dans le système réactionnel, il est possible de faire usage des réacteurs continus conventionnels à lit fixe (trickle-bed) ou à slurry. Dans le cas d'un réacteur discontinu, on fait généralement usage d'un autoclave muni d'un agitateur à pales.

Le procédé selon l'invention est particulièrement bien adapté à la synthèse de tert-amylbenzène par alkylation de cumène avec de l'éthylène.

Les exemples qui suivent ont pour but d'illustrer l'invention sans en limiter sa portée.

### Exemples 1R à 3R (selon l'art antérieur)

Dans un autoclave d'une contenance de 400 ml muni d'un agitateur à pales, on a introduit 86 g de cumène, 0,43 g de sodium métallique en dispersion dans la paraffine et 5 g d'un support préalablement séché par chauffage à 200°C et choisi parmi la silice, la zéolithe Y et le noir de carbone.

L'autoclave a ensuite été fermé et chauffé à 100°C pendant 15 minutes sous agitation et purge d'azote. On a ensuite chauffé le mélange réactionnel jusqu'à 185°C et on a démarré la réaction en injectant de l'éthylène sous une pression de 4,1 MPa dans le réacteur.

On a laissé la réaction se poursuivre pendant 2,5 heures, après quoi on a dosé le tert-amylbenzène formé et le cumène résiduel. Les résultats ont été portés au tableau 1 qui suit.

**Tableau 1**

| N° de l'exemple | 1R | 2R | 3R |
|---|---|---|---|
| Nature du support du catalyseur | Silice | Zéolithe Y | Noir de C |
| Taux de conversion du cumène, % | <1 | <1 | <1 |
| Sélectivité par rapport au cumène, % | 99 | 99 | 99 |
| Rendement en TAB par rapport au cumène, % | <1 | <1 | <1 |

Dans ce tableau, le taux de conversion par rapport au cumène est égal à 100 fois le rapport molaire du cumène consommé au cumène mis en oeuvre, la sélectivité par rapport au cumène est égale à 100 fois le rapport molaire du tert-amylbenzène (TAB) formé au cumène consommé et le rendement par rapport au cumène est égal à 100 fois le rapport molaire du tert-amylbenzène formé au cumène mis en oeuvre, c'est-à-dire au produit des deux rapports molaires précédents.

On voit que les procédés de l'art antérieur appliqués à la synthèse du tert-amylbenzène sont inopérants.

### Exemple 4 (selon l'invention)

On a reproduit l'exemple 1 en remplaçant le support de silice par une alumine γ, de marque SPHERALITE et de type SCS79, fabriquée par la Société RHONE-POULENC, qui présentait les caractéristiques suivantes :
- surface spécifique, m²/g 80
- volume de pores total, ml/g 0,6
- diamètre moyen des pores, nm 30

Les résultats obtenus ont été les suivants :
- taux de conversion du cumène, % 30,8
- sélectivité par rapport au cumène, % 99
- rendement en TAB par rapport au cumène, % 30,5

Ils dénotent de façon claire la supériorité du procédé selon l'invention.

### Exemples 5 à 9 (selon l'invention)

On a reproduit l'exemple 4 en mettant en oeuvre diverses qualités d'alumines γ et en modifiant les conditions opératoires suivantes :
- quantité de sodium : 0,86 g
- quantité de support : 10 g

Les alumines γ utilisées étaient des alumines de marque SPHERALITE, fabriquées par la société RHONE-POULENC et présentaient les caractéristiques détaillées au tableau 2 qui suit :

**Tableau 2**

| Type de l'alumine | SCS 9 | SCS 79 | SCS 100 | SAS 350 | SAP 350 |
|---|---|---|---|---|---|
| Surface spécifique, m2/g | 8 | 80 | 100 | 350 | 350 |
| Volume de pores total, ml/100g | 45 | 60 | 50 | 45 | 23 |
| Diamètre moyen des pores, nm | 300 | 30 | 20 | 5 | 2,5 |

Les résultats obtenus ont été les suivants (tableau 3) :

**Tableau 3**

| N° de l'exemple | 5 | 6 | 7 | 8 | 9 |
|---|---|---|---|---|---|
| Type de l'alumine | SCS 9 | SCS 79 | SCS 100 | SAS 350 | SAP 350 |
| Taux de conversion du cumène, % | 4,7 | 97,0 | 99,3 | 31,7 | <1 |
| Sélectivité par rapport au cumène, % | 99 | 99 | 99 | 99 | 99 |
| Rendement en TAB par rapport au cumène, % | 4,7 | 96,0 | 98,3 | 31,4 | <1 |

On notera les très bons résultats obtenus avec les alumines SCS 79 et SCS 100.

### Exemples 10R à 13R (non conformes à l'invention)

On a reproduit l'exemple 4 en modifiant le catalyseur comme suit
- exemple 10R : on n'a mis en oeuvre que 5 g d'alumine de type SCS 79 en omettant le sodium;
- exemples 11R à 13R : on n'a mis en oeuvre que du sodium ou de l'hydrure de sodium sans le support d'alumine;
   - exemple 11R : 0,43 g de Na en dispersion dans la paraffine;
   - exemple 12R : 0,86 g de Na en dispersion dans la paraffine;
   - exemple 13R : 0,86 g de NaH en dispersion dans une huile minérale.

Les résultats obtenus sont donnés au tableau 4 qui suit.

**Tableau 4**

| N° de l'exemple | 10R | 11R | 12R | 13R |
|---|---|---|---|---|
| Nature du catalyseur | SCS 79 seule | Na seul | Na seul | NaH seul |
| Taux de conversion du cumène, % | <1 | <1 | 1,5 | <1 |
| Sélectivité par rapport au cumène, % | 99 | 99 | 99 | 99 |
| Rendement en TAB par rapport au cumène, % | <1 | <1 | 1,48 | <1 |

Ces résultats montrent que les catalyseurs non conformes à l'invention constitués uniquement du support d'alumine ou bien du métal alcalin ou d'un hydrure de ce métal sont totalement inefficaces.

### Exemples 14 à 17 (conformes à l'invention)

On a répété l'exemple 4 en faisant varier la quantité de sodium pour une même quantité d'alumine (10 g).

**Tableau 5**

| N° de l'exemple | 14 | 15 | 16 | 17 |
|---|---|---|---|---|
| Quantité de Na, g | 0,26 | 0,34 | 0,43 | 0,86 |
| Taux de conversion du cumène, % | 12,4 | 52,3 | 70,7 | 97,0 |
| Sélectivité par rapport au cumène, % | 99 | 99 | 99 | 99 |
| Rendement en TAB par rapport au cumène, % | 12,3 | 51,8 | 70,0 | 96,0 |

### Exemples 18 à 21

On a répété l'exemple 6 en remplaçant le sodium dispersé dans la paraffine par une quantité variable d'une dispersion d'hydrure de sodium dans une huile minérale.

Les résultats ont été portés dans le tableau 6 ci-après.

**Tableau 6**

| N° de l'exemple | 18 | 19 | 20 | 21 |
|---|---|---|---|---|
| Quantité de NaH, g | 0,1 | 0,2 | 0,27 | 1,13 |
| Taux de conversion du cumène, % | <1 | 93,6 | 99,4 | 99,8 |
| Sélectivité par rapport au cumène, % | 99 | 99 | 99 | 99 |
| Rendement en TAB par rapport au cumène, % | <1 | 92,7 | 98,4 | 98,8 |

On voit qu'une quantité d'hydrure de sodium aussi faible que 0,2 g est déjà suffisante pour obtenir des rendements en TAB supérieurs à 90 %.

### Exemples 22 à 25 (conformes à l'invention)

On a répété l'exemple 6 en faisant varier la température de la réaction.

Les résultats ont été les suivants :

**Tableau 7**

| N° de l'exemple | 22 | 23 | 24 | 25 |
|---|---|---|---|---|
| Température de l'essai, °C | 169 | 177 | 181 | 185 |
| Taux de conversion du cumène, % | <1 | 27,6 | 92,5 | 98,0 |
| Sélectivité par rapport au cumène, % | 99 | 99 | 99 | 99 |
| Rendement en TAB par rapport au cumène, % | <1 | 27,3 | 91,6 | 97,0 |

Les résultats obtenus montrent que la température optimale est proche de 185°C.

### Exemple 26 (conforme à l'invention)

On a reproduit l'exemple 6 en remplaçant l'éthylène par du propylène sous une pression de 0,8 MPa.
Après avoir laissé réagir pendant 4 heures sous agitation à 185°C, on a décelé dans le milieu réactionnel la présence de 2-méthyl,2-phénylpentane (MPP) et de 2,3-diméthyl,2-phénylbutane (DPB). Le dosage de ces produits a donné les résultats qui suivent
- Taux de conversion du cumène, % 15,2
- Sélectivité du MPP par rapport au cumène, 79
- Sélectivité du DPB par rapport au cumène, 21
- Rendement en MPP par rapport au cumène, 12,0
- Rendement en DPB par rapport au cumène, 3,2

### Exemples 27 à 30 (conformes à l'invention)

Dans un autoclave en acier inoxydable d'une capacité de 400 ml muni d'un système d'agitation à turbine tournant à 1000 tours/min, on a introduit 86 g de cumène, 10 g d'alumine de marque EXP 2001 P de la firme RHONE-POULENC et préalablement séchée par chauffage à 200 °C, 1 g de métal alcalin ou d'hydrure de métal alcalin et éventuellement 1 g de KOH. L'alumine EXP 2001 P possède les mêmes caractéristiques que l'alumine SCS 79 de l'exemple 6.

L'autoclave a ensuite été fermé et chauffé à 185 °C pendant 15 minutes sous agitation et purge d'azote. On a ensuite injecté de l'éthylène sous une pression de 4 MPa dans l'autoclave. Dès que la réaction démarre on maintient la température, la pression et l'agitation pendant 60 minutes.

Les résultats obtenus figurent au tableau 8 qui suit :

**Tableau 8**

| N° de l'exemple | 27 | 28 | 29 | 30 |
|---|---|---|---|---|
| Type de catalyseur | NaH | NaH | Na | K |
| Teneur en KOH, % | 0 | 1 | 1 | 1 |
| Taux de conversion du cumène, % | 95,2 | 99,0 | 94,2 | 99,4 |
| Période d'induction, min | 30 | 0 | 12 | 0 |

La période d'induction correspond à l'intervalle de temps qui s'est écoulé entre le moment de l'injection d'éthylène et le démarrage de la réaction. Dans le cas des essais 28 et 30, la réaction a été beaucoup plus rapide et a été arrêtée après 15 minutes au lieu de 60 minutes.
La sélectivité de la réaction par rapport au cumène a été pour chacun des exemples 27 à 30 d'environ 99 %.

### Exemples 31 et 32 (conformes à l'invention)

On a procédé comme aux exemples 27 à 30 excepté que la quantité d'alumine a été réduite à 2 g et celle d'hydrure de sodium à 0,2 g. Les deux essais ont été effectués en présence de 0,2 g de KOH lors de la préparation du catalyseur. L'exemple 31 a été réalisé avec l'alumine de marque EXP 2001 P, l'exemple 32 avec l'alumine EXP 531 P, toutes deux fabriquées par la firme RHONE-POULENC et de caractéristiques physiques semblables.

Les résultats obtenus ont été les suivants :

| Exemple N° | Temps de réaction, min | Période d'induction, min | Taux de conversion du cumène, % |
|---|---|---|---|
| 31 | 60 | 3 | 98,4 |
| 32 | 60 | 0 | 97,1 |

La sélectivité de la réaction par rapport au cumène a été dans les deux cas d'environ 99 %.

### Exemples 33R et 34R

On a répété l'exemple 31 mais en traitant l'alumine au préalable pendant 2 heures à 500 °C sous atmosphère d'azote dans un four tournant. Après refroidissement à 350 °C, on a traité l'alumine au moyen de KOH à raison de 10 % de son poids (exemple 33R) et de 5 % de son poids (exemple 34R) dans le même four tournant pendant une durée de 3 heures et on a ensuite laissé refroidir à température ambiante. On a ensuite poursuivi comme dans l'exemple 31 en mettant en oeuvre, dans le milieu réactionnel, 2 g d'alumine traitée et 0,2 g d'hydrure de sodium sans addition de KOH dans le milieu.

Les résultats obtenus ont été les suivants :

| Exemple N° | Temps de réaction, min | Période d'induction, min | Taux de conversion du cumène, % |
|---|---|---|---|
| 33R | 60 | > 180 | 0 |
| 34R | 60 | 90 | 41,3 |

### Exemples 35R et 36R

On a répété l'exemple 31 mais en remplaçant le K0H par du Na0H lors de la préparation du catalyseur à raison de 0,2 g (exemple 35R) et de 0,1 g (exemple 36R).

Les résultats obtenus ont été les suivants :

| Exemple N° | Temps de réaction, min | Période d'induction, min | Taux de conversion du cumène, % |
|---|---|---|---|
| 35R | 60 | 150 | 28,8 |
| 36R | 60 | 114 | 20,5 |

### Exemples 37R et 38R

On a reproduit les exemples 33R et 34R excepté qu'on a fait suivre le traitement de l'alumine par du ROH par un traitement supplémentaire de l'alumine par du potassium métallique à raison de 10 % du poids de l'alumine de départ. On a poursuivi le traitement par le potassium pendant 30 minutes à 290 °C et on a ensuite refroidi à température ambiante le catalyseur ainsi préparé. Dans l'exemple 37R, on a mis en oeuvre 2 g de catalyseur, dans l'essai 38R, on a réduit à 0,4 g la quantité de catalyseur.

Les résultats obtenus ont été les suivants :

| Exemple N° | Temps de réaction, min | Période d'induction, min | Taux de conversion du cumène, % |
|---|---|---|---|
| 37R | 60 | 0 | 94,5 |
| 38R | 60 | > 180 | 0 |

## Revendications

1. Procédé de synthèse d'hydrocarbures aromatiques alkylés à chaîne alkyle saturée comprenant au moins quatre atomes de carbone selon lequel on fait réagir un hydrocarbure aromatique substitué par un groupement alkyle à chaîne courte saturée comportant un à trois atomes de carbone avec une oléfine en présence d'un catalyseur constitué d'au moins un métal alcalin ou un hydrure de métal alcalin imprégné sur un support d'alumine caractérisé en ce que l'on prépare le catalyseur dans le milieu réactionnel en présence de l'hydrocarbure aromatique à courte chaîne alkyle par mélange de l'alumine anhydre avec le métal alcalin ou avec l'hydrure de métal alcalin, puis on introduit l'oléfine pour démarrer la réaction.

2. Procédé selon la revendication 1, caractérisé en ce que lors de la préparation du catalyseur on ajoute de l'hydroxyde de potassium au milieu réactionnel.

3. Procédé selon la revendication 1 ou 2, caractérisé en ce que le support d'alumine présente un diamètre moyen de pores compris entre 2 et 100 nm.

4. Procédé selon l'une quelconque des revendications 1 à 3, caractérisé en ce que le support d'alumine est constitué par de l'alumine γ.

5. Procédé selon l'une quelconque des revendications 1 à 4, caractérisé en ce que le support d'alumine présente un volume de pores compris entre 25 et 65 ml/100 g d'alumine et une surface spécifique comprise entre 10 et 360 m²/g d'alumine.

6. Procédé selon l'une quelconque des revendications 1 à 5, caractérisé en ce que le rapport pondéral du métal alcalin ou de l'hydrure de métal alcalin au support d'alumine est compris entre 0,02 et 0,50.

7. Procédé selon l'une quelconque des revendications 1 à 6, caractérisé en ce que le rapport pondéral du métal alcalin ou de l'hydrure de métal alcalin à l'hydrocarbure aromatique à courte chaîne est situé dans la plage allant de 0,0015 à 0,03.

8. Procédé selon l'une quelconque des revendications 1 à 7, caractérisé en ce que le métal alcalin est du sodium et l'hydrure de métal alcalin de l'hydrure de sodium.

9. Procédé selon l'une quelconque des revendications 1 à 8, caractérisé en ce qu'on effectue la réaction à une température comprise entre 170 et 190°C.

10. Utilisation du procédé selon l'une quelconque des revendications 1 à 9 pour la synthèse du tert-amylbenzène par alkylation de cumène avec de l'éthylène.

## Patentansprüche

1. Verfahren zur Synthese von aromatischen Kohlenwasserstoffen, die mit einer gesättigten Alkylkette, die wenigstens vier Kohlenstoffatome umfaßt, alkyliert sind, gemäß dem man einen aromatischen Kohlenwasserstoff, der mit einer Alkylgruppe mit kurzer gesättigter Kette, die ein bis drei Kohlenstoffatome umfaßt, substituiert ist, mit einem Olefin in Gegenwart eines Katalysators, der aus wenigstens einem Alkalimetall oder einem Alkalimetallhydrid aufgebracht auf einem Aluminiumoxidträger besteht, umsetzt, dadurch gekennzeichnet, daß man den Katalysator im Reaktionsmedium in Gegenwart des aromatischen Kohlenwasserstoffs mit kurzer Alkylkette durch Mischen von wasserfreiem Aluminiumoxid mit dem Alkalimetall oder mit dem Alkalimetallhydrid herstellt, man dann das Olefin zugibt, um die Reaktion zu starten.

2. Verfahren gemäß Anspruch 1, dadurch gekennzeichnet, daß man bei der Herstellung des Katalysators Kaliumhydroxid zum Reaktionsmedium zusetzt.

3. Verfahren gemäß Anspruch 1 oder 2, dadurch gekennzeichnet, daß der Aluminiumoxidträger einen mittleren Porendurchmesser zwischen 2 und 100 nm aufweist.

4. Verfahren gemäß einem der Ansprüche 1 bis 3, dadurch gekennzeichnet, daß der Aluminiumoxidträger aus γ-Aluminiumoxid besteht.

5. Verfahren gemäß einem der Ansprüche 1 bis 4, dadurch gekennzeichnet, daß der Aluminiumoxidträger ein Porenvolumen zwischen 25 und 65 ml/100 g Aluminiumoxid und eine spezifische Oberfläche zwischen 10 und 360 m²/g Aluminiumoxid aufweist.

6. Verfahren gemäß einem der Ansprüche 1 bis 5, dadurch gekennzeichnet, daß das Gewichtsverhältnis des Alkalimetalls oder des Alkalimetallhydrids zu dem Aluminiumoxidträger zwischen 0,02 und 0,50 liegt.

7. Verfahren gemäß einem der Ansprüche 1 bis 6, dadurch gekennzeichnet, daß das Gewichtsverhältnis des Alkalimetalls oder des Alkalimetallhydrids zu dem kurzkettigen aromatischen Kohlenwasserstoff in dem Bereich von 0,0015 bis 0,03 liegt.

8. Verfahren gemäß einem der Ansprüche 1 bis 7, dadurch gekennzeichnet, daß das Alkalimetall Natrium und das Alkalimetallhydrid Natriumhydrid ist.

9. Verfahren gemäß einem der Ansprüche 1 bis 8, dadurch gekennzeichnet, daß man die Reaktion bei einer Temperatur zwischen 170 und 190 °C ausführt.

10. Verwendung des Verfahrens gemäß einem der Ansprüche 1 bis 9 für die Synthese von tert.-Amylbenzol durch Alkylierung von Cumol mit Ethylen.

## Claims

1. Process for the synthesis of alkylated aromatic hydrocarbons with a saturated alkyl chain containing at least four carbon atoms, according to which an aromatic hydrocarbon substituted by a saturated short-chain alkyl group containing one to three carbon atoms is reacted with an olefin in the presence of a catalyst consisting of at least one alkali metal or an alkali metal hydride impregnated onto an alumina support, characterized in that the catalyst is prepared in the reaction medium in the presence of the aromatic hydrocarbon with a short alkyl chain by mixing anhydrous alumina with the alkali metal or with the alkali metal hydride, and the olefin is then introduced to start the reaction.

2. Process according to Claim 1, characterized in that potassium hydroxide is added to the reaction mixture during the catalyst preparation.

3. Process according to Claim 1 or 2, characterized in that the alumina support has a mean pore diameter of between 2 and 100 nm.

4. Process according to any one of Claims 1 to 3, characterized in that the alumina support consists of γ alumina.

5. Process according to any one of Claims 1 to 4, characterized in that the alumina support has a pore volume of between 25 and 65 ml/100 g of alumina and a specific surface of between 10 and 360 m²/g of alumina.

6. Process according to any one of Claims 1 to 5, characterized in that the weight ratio of the alkali metal or of the alkali metal hydride to the alumina support is between 0.02 and 0.50.

7. Process according to any one of Claims 1 to 6, characterized in that the weight ratio of the alkali metal or of the alkali metal hydride to the short-chain aromatic hydrocarbon lies in the range from 0.0015 to 0.03.

8. Process according to any one of Claims 1 to 7, characterized in that the alkali metal is sodium and the alkali metal hydride is sodium hydride.

9. Process according to any one of Claims 1 to 8, characterized in that the reaction is performed at a temperature of between 170 and 190°C.

10. Use of the process according to any one of Claims 1 to 9 for the synthesis of tert-amylbenzene by alkylation of cumene with ethylene.
